# EUROPÄISCHE PATENTSCHRIFT

(11) **EP 1 685 863 B1**
(45) Veröffentlichungstag und Bekanntmachung des Hinweises auf die Patenterteilung: **21.04.2010**
(21) Anmeldenummer: 04788528.0
(22) Anmeldetag: 20.09.2004
(51) Int. Cl.: A61M 1/36, A61M 1/00, B03C 1/28

(54) **SYSTEM ZUR KORREKTUR VON BIOLOGISCHER FLÜSSIGKEIT**
SYSTEM FOR CORRECTING BIOLOGICAL FLUID
SYSTEME DE PURIFICATION D'UN LIQUIDE BIOLOGIQUE

(30) Priorität: 14.10.2003 RU 2003130214
(43) Veröffentlichungstag der Anmeldung: 02.08.2006
(73) Patentinhaber: Kutushov, Mikhail Vladimirovich, Moscow, 125414 (RU); Germanov, Evgeny Pavlovich, Moscow, 121085 (RU)
(72) Erfinder: KUTUSHOV, Mikhail Vladimirovich, Moscow, 125414 (RU)
(74) Vertreter: Jeck, Anton
(86) Internationale Anmeldenummer: PCT/RU2004/000367
(87) Internationale Veröffentlichungsnummer: WO 2005/035024

(56) Entgegenhaltungen:
- WO-A-90/04019
- WO-A1-01/24850
- WO-A1-94/21310
- DE-A1- 10 062 833
- DE-A1- 19 917 522
- RU-U1- 1 430
- SU-A3- 1 836 105
- US-A- 3 774 611
- US-A- 5 980 479
- US-B1- 6 616 623

## Beschreibung

Die Erfindung betrifft ein Korrektursystem der Körperflüssigkeit mit
- luftdichten, mit Rückschlagventilen gekoppelten Durchläufen, wobei die Rückschlagventile so installiert sind, dass sie ein Durchströmen der Körperflüssigkeit von einem Eingangsrohransatz zu einem Ausgangsrohransatz ermöglichen,
- einer Vermischungskammer zur Vermischung eines magnetisch gesteuerten Sorbens mit der Körperflüssigkeit,
- einer Ausscheidungskammer zur Ausscheidung dieses magnetisch gesteuerten Sorbens aus dieser Körperflüssigkeit,
- einem luftdichten Behälter für das magnetisch gesteuerte Sorbens und
- einem Filtergerät, das mit dem Durchlauf der Ausscheidungskammer und dem Ausgangsrohransatz des Systems gekoppelt ist.

Die vorliegende Erfindung gehört zur Biologie und Medizin und kann für die Reinigung von Körperflüssigkeiten und für die Anpassung der Zusammensetzung der Körperflüssigkeit nach physiologischen Normen eingesetzt werden.

Es ist ein Gerät zur Korrektur von Körperflüssigkeit bekannt (s. beispielsweise die WO 94 213 10 A1; 1994). Das Gerät enthält eine Vermischungskammer zur Vermischung der Körperflüssigkeit (im Weiteren kurz Vermischungskammer genannt) mit einem magnetisch gesteuerten Sorbens (russisch MUS, deutsch MGS), das beispielsweise in einer physiologischen Lösung vorhanden ist. Das Gerät enthält auch eine Ausscheidungskammer (im Weiteren kurz Ausscheidungskammer genannt) zur Ausscheidung des magnetisch gesteuerten Sorbens aus der Körperflüssigkeit mit Hilfe von Magneten nach dem Wechselwirkungsvorgang der Körperflüssigkeit mit dem magnetisch gesteuerten Sorbens. Das Gerät enthält auch einen Behälter (im Weiteren kurz Behälter genannt) für das magnetisch gesteuerte Sorbens mit der physiologischen Lösung sowie einen Antrieb, der den Gerätelauf sicherstellt.

Die Vermischungskammer ist durch Durchläufe mit dem Behälter und der Ausscheidungskammer verbunden, die durch ein Filtergerät an einen Ausgangsrohransatz des Korrekturgeräts und durch einen Eingangsrohransatz an die Einströmquelle der Körperflüssigkeit (beispielsweise an die Blutader eines Patienten) angeschlossen ist. Dabei ist der Eingangsrohransatz durch einen Durchlauf mit der Vermischungskammer verbunden, und in denselben Durchlauf wurde ein Ausgangsdurchlauf des Behälters eingeleitet. Außerdem wurden in den Durchläufen Ventilverschlüsse installiert, die die Körperflüssigkeitsbewegung vom Eingangsrohransatz des Gerätes zum Ausgangsrohransatz ermöglichen.

Das bekannte Gerät ermöglicht es, die Körperflüssigkeit durch Entfernung von beispielsweise nieder- und mittelmolekularen Toxinen zu reinigen, aber für diesen Einsatz muss die zu korrigierende Flüssigkeit mit der physiologischen Lösung vermischt werden, und es müssen der zu korrigierenden Flüssigkeit (beispielsweise Blut) Antigerinnungsmittel zugesetzt werden. Dies sagt einem Patienten nicht immer zu. Außerdem ist der Aufbauvorgang ganz kompliziert.

Ein ist das Korrektursystem für die Körperflüssigkeit, s. beispielsweise das Patent US 5 980 479, mit der Priorität vom 2. Juli 1997, das eine luftdichte Vermischungskammer, eine luftdichte Ausscheidungskammer und einen luftdichten Behälter für das magnetisch gesteuerte Sorbens enthält. Dabei ist die Vermischungskammer mit Durchlaufschläuchen mit dem Behälter und mit einer Reinigungskammer verbunden, die durch ein Filtergerät an den Ausgangsrohransatz des Korrekturgeräts und durch einen Eingangsrohransatz an die Einströmquelle der Körperflüssigkeit (beispielsweise an die Blutader eines Patienten) angeschlossen ist.

Die Bewegung der Körperflüssigkeit vom Eingangsrohransatz des Geräts zum Ausgangsrohransatz ermöglichen auf den Durchläufen installierte Pumpen, wobei der Eingangsrohransatz mit dem Durchlauf an die Vermischungskammer angeschlossen und in diesen Durchlauf der Ausgangsdurchlauf des Behälters eingeleitet ist.

Außerdem sind in den Durchläufen Ventilverschlüsse installiert, die die angegebene Bewegungsrichtung der Körperflüssigkeit kontrollieren. Der Behälter ist mit einem Gerät für die Aufrechterhaltung des angegebenen Drucks in der Körperflüssigkeit ausgestattet.

Ein solches System ermöglicht die Reinigung der Körperflüssigkeit, doch dieses System weist die oben genannten Mängel des behandelten Geräts auf. Außerdem ist die Möglichkeit nicht ausgeschlossen, dass Luft in die zu bearbeitende Körperflüssigkeit eindringt, mit deren Hilfe beispielsweise der angegebene Druck im Behälter mit dem magnetisch gesteuerten Sorbens in der physiologischen Lösung aufrechterhalten wird. Der Systemaufbau des Geräts, das die bearbeitete Flüssigkeit vom Geräteauslass aus dem System filtriert, ist sehr kompliziert.

Die Aufgabe der Erfindung besteht darin, eine technische Lösung zu entwickeln, die die Reinigung der Körperflüssigkeit bei einer Kleinstmenge der darin eingeleiteten Fremdreagenzien ermöglicht.

Die gestellte Aufgabe wird durch die Merkmale des Anspruchs 1 gelöst.

Es ist ein luftdichter Behälter für das magnetisch gesteuerte Sorbens, eine luftdichte Vermischungskammer für die Vermischung des magnetisch gesteuerten Sorbens mit der Körperflüssigkeit und eine Ausscheidungskammer zur Ausscheidung des magnetisch gesteuerten Sorbens aus dieser Flüssigkeit sowie ein Filtergerät im Korrektursystem der Körperflüssigkeit vorgesehen. Das Korrektursystem enthält mit Durchläufen verbundene Rückschlagventile, die darin installiert sind, wobei sie den Durchlass durch das Körperflüssigkeitssystem vom Eingangsrohransatz zum Ausgangsrohransatz ermöglichen. Das Filtergerät ist durch den Ausgangsdurchlauf des Systems mit dem Ausgangsrohransatz verbunden, der mit dem Eingangsdurchlauf des Systems verbunden ist. Die Vermischungskammer und die Ausscheidungskammer zur Ausscheidung des magnetisch gesteuerten Sorbens sowie der Behälter für das magnetisch gesteuerte Sorbens sind derart ausgebildet, dass ihre Volumina verändert werden können. Sie sind mit einem entsprechenden Antrieb ausgestattet, wobei die Vermischungskammer zur Vermischung des magnetisch gesteuerten Sorbens mit der Körperflüssigkeit und die Ausscheidungskammer zur Ausscheidung des magnetisch gesteuerten Sorbens aus dieser Flüssigkeit als Behälter ausgebildet sind, die einen gemeinsamen Deckel und eine an den gemeinsamen Boden dieser Kammern anschließende Wand aufweisen und die fest miteinander verbunden sind. Die Wand ist als Zwischenkammertrennwand ausgebildet, wobei die Hohlräume der Kammern durch den Durchlauf in dieser Wand verbunden sind und Rillen an anderen Seitenwänden dieser Kammern angebracht sind, die entsprechende Zylinder Faltrohre bilden.

Die Deckel der Kammern sind gelenkig mit der Drehungsmöglichkeit um den Gelenkdrehpunkt an deren gemeinsamer Wand befestigt. Dabei ist der Behälter für das magnetisch gesteuerte Sorbens innerhalb der Vermischungskammer zur Vermischung des magnetisch gesteuerten Sorbens mit der Körperflüssigkeit installiert und mit als Zylinder - Faltrohr geriffelten Seitenfläche ausgebildet, wobei eine Stirnseite dieses Zylinders am Boden der Vermischungskammer zur Vermischung des magnetisch gesteuerten Sorbens mit der Körperflüssigkeit befestigt ist. An der anderen Stirnseite ist ein Deckel installiert, der auf dem Deckel dieser Kammer befestigt ist. Dabei sind Magneten auf dem Boden der Abscheidungskammer des magnetisch gesteuerten Sorbens aufgestellt, und der Eingangsrohransatz des Systems ist gleichzeitig mit den Innenräumen der Vermischungskammer des magnetisch gesteuerten Sorbens und des Behälters für das magnetisch gesteuerte Sorbens verbunden, wobei der Behälter mit dem Innenraum der Vermischungskammer des magnetisch gesteuerten Sorbens verbunden ist.

Außerdem sind die Deckel der Vermischungskammer und der Ausscheidungskammer des magnetisch gesteuerten Sorbens miteinander verbunden oder befinden sich auf einer Ebene oder sind in der Seitenansicht im Schnitt V-förmig ausgebildet. Das mit der Vermischungskammer und mit der Ausscheidungskammer gebildete Gehäuse ist im Grundriss entweder als Rechteck mit gerundeten Winkeln oder als Kreis, entweder als Oval oder als Ziffer 8 ausgebildet. Dabei sind die Volumina der Innenräume der Vermischungskammer zur Vermischung des magnetisch gesteuerten Sorbens und der Ausscheidungskammer zur Ausscheidung des magnetisch gesteuerten Sorbens im Verhältnis von 1:1, oder von 1:(0,1-0,9), oder von (0,1-0,9): 1 entsprechend ausgewählt. Das Volumen der Innenräume der Vermischungskammer zur Vermischung des magnetisch gesteuerten Sorbens und des Behälters für das magnetisch gesteuerte Sorbens sind im Verhältnis von 1:(0,1-0,9) ausgewählt. Außerdem ist der Behälter für das magnetisch gesteuerte Sorbens in der Vermischungskammer zur Vermischung des magnetisch gesteuerten Sorbens mit einem Abstand von der Seitenwand dieser Kammer von mindestens (1-100)d und mit einem Abstand von der Wand zwischen der Vermischungskammer und der Abscheidungskammer von mindestens (10-100)d installiert. Dabei stellt d den Innendurchmesser des Durchlaufs dar, der den Eingangsrohransatz des Systems mit dem Innenraum der Vermischungskammer zur Vermischung des magnetisch gesteuerten Sorbens verbindet.

Dabei ist der Durchlauf vom Eingangsrohransatz in die Vermischungskammer zur Vermischung des magnetisch gesteuerten Sorbens entweder durch den Boden oder durch den Deckel der Kammer eingeleitet. Der Durchlauf des Eingangsrohransatzes ist in die Vermischungskammer unter einem Winkel von 10-80° zur Bodenfläche oder entsprechend zum Deckel der Kammer und zur Senkrechten eingeleitet. Der Durchlauf vom Eingangsrohransatz ist in den Behälter für das magnetisch gesteuerte Sorbens durch den Deckel des Behälters oder durch dessen Boden eingeleitet, und der Durchlauf aus dem Behälter für das magnetisch gesteuerte Sorbens ist in der Vermischungskammer beispielsweise im Unterteil der Seitenwand des Behälters zur Ausscheidung des magnetisch gesteuerten Sorbens mit einem Abstand vom Kammerboden von (0,5-50)d installiert, wobei d der Durchmesser des Durchlaufs ist.

Außerdem ist der Durchlauf zwischen der Vermischungskammer zur Vermischung des magnetisch gesteuerten Sorbens und der Ausscheidungskammer zur Ausscheidung des magnetisch gesteuerten Sorbens in der Wand zwischen den Kammern mit einem Abstand vom Boden der Kammer von (0,5-50)d installiert, wobei d der Durchmesser des Durchlaufs ist. Der Durchlauf aus der Ausscheidungskammer ist im Obenteil der Seitenwand der Kammer mit einem Abstand vom Deckel von (0,5-50)d installiert, wobei d der Durchmesser des Durchlaufs ist.

Die Magnete sind entweder innerhalb der Ausscheidungskammer zur Ausscheidung des magnetisch gesteuerten Sorbens oder außerhalb der Kammer aufgestellt und sind auf dem Boden der Ausscheidungskammer befestigt.

Außerdem ist der Antrieb zur Volumenveränderung der Vermischungskammer zur Vermischung des magnetisch gesteuerten Sorbens und der Ausscheidungskammer zur Ausscheidung des magnetisch gesteuerten Sorbens sowie des Behälters für das magnetisch gesteuerte Sorbens als Elektromotor ausgebildet, der mit dem Deckel entweder durch einen Räderkasten oder durch einen Kurventrieb verbunden oder als auf der Abtriebswelle befestigter Radkasten unter einem Winkel von 30-45° zur Walzenachse eines Scheibchens ausgebildet ist, das bei der Walzenumdrehung abwechselnd mit den Deckeln der Kammern zusammenwirkt. Der Antrieb kann auch als ein mit dem Deckel verbundener Kurventrieb ausgebildet sein, dessen Lauf die Handsteuerung einer Bedienungsperson ermöglicht. Dieser Antrieb kann auch so gefertigt werden, dass dessen Lauf die Handsteuerung des Deckels unmittelbar ermöglicht.

Dabei wird als ein Stellort auf dem Deckel eine Stelle über der geriffelten Seitenwand der Vermischungskammer oder über der geriffelten Seitenwand der Ausscheidungskammer zur Ausscheidung des magnetisch gesteuerten Sorbens ausgewählt.

Außerdem sind die Durchmesser der Rohransätze in die Vermischungskammer und in den Behälter für das magnetisch gesteuerte Sorbens im Verhältnis von d/d₁=V/V₁ ausgewählt, wobei d den Innendurchmesser des Rohransatzes in die Vermischungskammer, d₁ den Innendurchmesser des Rohransatzes in den Behälter für das magnetisch gesteuerte Sorbens, V das Volumen der Vermischungskammer und V₁ das Volumen des Behälters für das magnetisch gesteuerte Sorbens darstellen.

Dabei sind die Wandungen des Behälters für das magnetisch gesteuerte Sorbens und der Vermischungskammer und der Ausscheidungskammer sowie die Wand zwischen diesen Kammern und der Deckel und der Boden beispielsweise aus Polyurethan hergestellt, und die Wellung macht (0,5-0,95)% der Höhe der entsprechenden Wand aus.

### Kurzbeschreibung der Zeichnungen

Die Erfindung wird nun anhand von Ausführungsbeispielen näher erläutert. Es zeigen:
- Fig. 1: ein Schema eines Korrektursystems der Körperflüssigkeit,
- Fig. 2: ein Schema eines Filtergeräts des Systems der Fig. 1,
- Fig. 3: eine Ansicht eines Systems mit V-artig verbundenen Deckeln,
- Fig. 4: eine Schemavariante eines Antriebs für eine Voluminaveränderung,
- Fig. 5: ein Schema einer Gelenkstütze des Kammernbodens des Systems,
- Figuren 6, 7 und 8: Darstellungsvarianten des Systems als Kreis, Oval oder Ziffer 8.

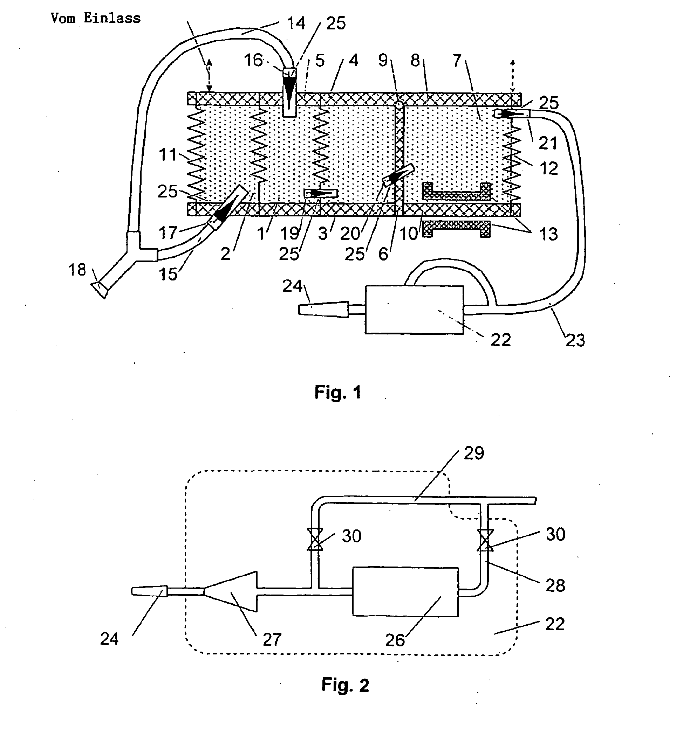

Das Korrektursystem der Körperflüssigkeit enthält gemäß Fig. 1 einen Behälter 1 zur Unterbringung der zu reinigenden Körperflüssigkeit, beispielsweise des Bluts eines Patienten, und zwar zur Reinigung von nieder- und mittelmolekularen Toxinen eines magnetisch gesteuerten Sorbens (in der Figur nicht dargestellt, s. jedoch beispiels-weise die WO 9421310 A1, 1994). Der Behälter 1 ist als Zylinderfaltrohr ausgebildet, das in einer Vermischungskammer 2 zur Vermischung des magnetisch gesteuerten Sorbens mit der Körperflüssigkeit installiert ist, die für Sicherung der Zusammenwirkung des magnetisch gesteuerten Sorbens mit der Körperflüssigkeit bestimmt ist. Dabei ist das Zylinderfaltrohr mit entsprechenden Rillen versehen (in der Figur nicht durchnummeriert). Die dadurch gebildete Wellung ist auf (0,5-0,95)% der Fläche (der Höhe nach) des Zylinderfaltrohrs vorgesehen. Mit einer Stirnfläche (in der Figur nicht durchnummeriert), neben der es keine Wellung gibt, ist der Behälter 1 auf einem Boden 3 der Vermischungskammer 2 befestigt. Die andere Stirnfläche dieses Behälters 1 ist auf einem Deckel 4 der Vermischungskammer 2 befestigt und hermetisch mit einem Deckel 5 zugedeckt.

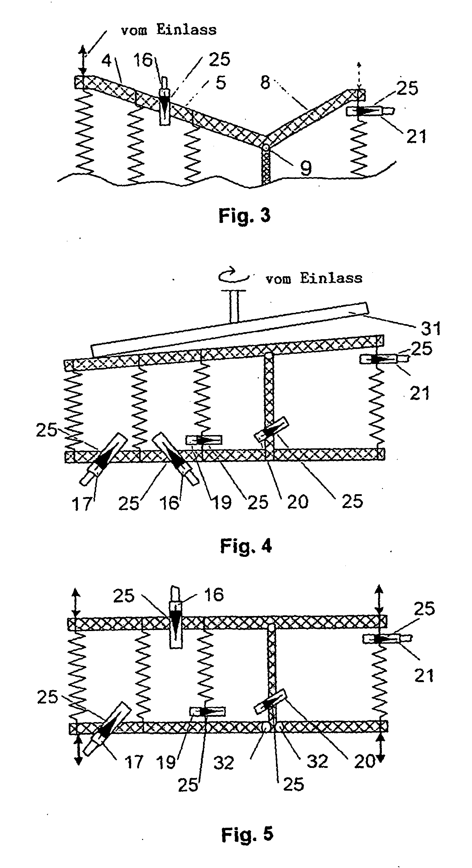

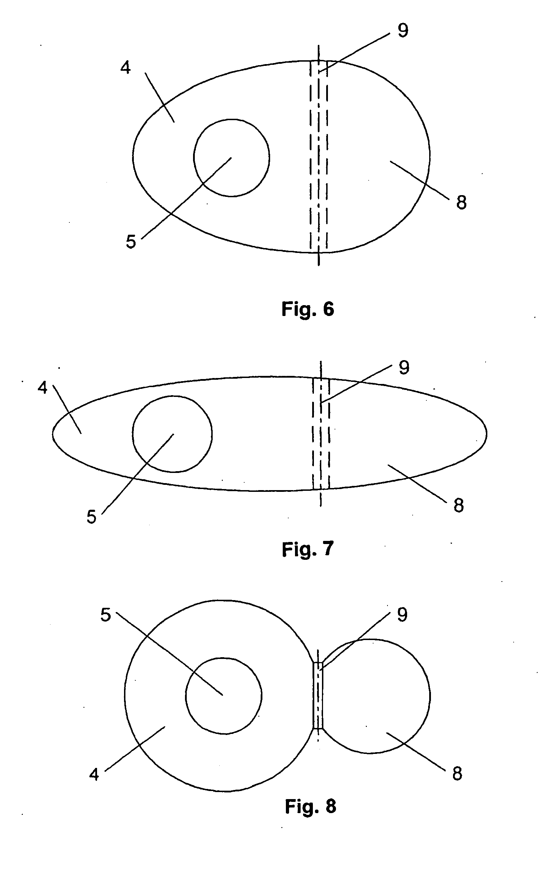

Der Boden 3 der Vermischungskammer 2 ist gemäß der Fig. 1 fest mit einer Wand 6 gekoppelt oder gemäß der Fig. 7 an der Wand 6 angelenkt, die als Zwischenkammer-Trennwand zwischen der Vermischungskammer 2 und der Ausscheidungskammer 7 zur Ausscheidung des magnetisch gesteuerten Sorbens dient, die ihrerseits zur Ausscheidung dieses Sorbens aus der Körperflüssigkeit bestimmt ist. Dabei sind der Deckel 4 der Vermischungskammer 2 und der Deckel 8 der Ausscheidungskammer 7 fest miteinander gekoppelt und an der Wand 6 mit einem Gelenk 9 unter der Voraussetzung des Vorhandenseins der Umdrehungsmöglichkeit um dieses Gelenk in der Fläche installiert (und zwar in der Fläche, die der Gelenkachse gegenüber lotrecht liegt; in der Figur nicht dargestellt). Dabei liegen die Deckel 4 und 8 gemäß Fig. 1 in einer Fläche oder gemäß Fig. 3 unter einem Winkel, beispielsweise als V-förmiger Schnitt. Die Deckelgrößen in diesem Schnitt (die Schenkelgröße des Buchstabens V) und dementsprechend die Winkelgröße zwischen ihnen sind unter Berücksichtigung der Sicherung des nötigen Verhältnisses des Volumens der Vermischungskammer 2 und des Volumens der Ausscheidungskammer 7 ausgewählt. Die Achse des Gelenks 9 ist im Kreuzpunkt dieser Schenkel platziert. Der Boden 10 der Ausscheidungskammer 7 und der Kreuzpunkt 3 der Vermischungskammer 2 sind gemäß Fig. 1 fest mit der Wand 6 gekoppelt oder gemäß Fig. 7 an dieser Wand angelenkt. Die Außenwände 11 und 12 der Vermischungskammer 2 und der Ausscheidungskammer 7 sind als geriffelte Zylinderfaltrohre gefertigt. Die Wellung ist sowohl im Behälter 1 für das magnetisch gesteuerte Sorbens als auch in der Vermischungskammer 2 und in der Ausscheidungskammer 7 auf (0,5-0,95)% der Höhe der entsprechenden Wände aufgetragen.

Die Böden 3 und 10, die Deckel 4, 5 und 8, die Wände 6, 11 und 12 der Vermischungskammer 2 und der Ausscheidungskammer 7 sowie die Wände des Behälters 1 (nicht dargestellt) sind aus antimagnetischen Stoffen, beispielsweise aus Polyurethan, hergestellt.

Auf dem Boden 10 der Ausscheidungskammer 7 sind Magnete 13 aufgestellt, die als Permanentmagnete aus einer Samarium(8t)-Kobalt(Co)-Legierung hergestellt sind. Sie dienen zur Ausscheidung des magnetisch gesteuerten Sorbens aus der Körperflüssigkeit, die mit diesem gemischt ist, wobei diese Magnete in Abhängigkeit von Baugründen oder zur Gewinnung des nötigen Werts des Magnetfelds entweder innerhalb dieser Kammer 7 unter einem Metallgitter (nicht dargestellt) oder an der Außenseite des Bodens 10 oder sowohl innerhalb der Kammer 7 als auch außerhalb dieser Kammer 7 aufgestellt werden können. Dabei muss die von ihnen erzeugte Stärke des Magnetfelds 10-200 MTesla betragen. Bei dem Beispiel gemäß der Fig. 1 wurde die Aufstellung der Magnete 13 sowohl innerhalb dieser Kammer 7 auf dem Boden 10 als auch an der Außenseite des Bodens 10 der Ausscheidungskammer 7 zur Ausscheidung des magnetisch gesteuerten Sorbens vorgenommen.

Der Behälter 1 für das magnetisch gesteuerte Sorbens und die Vermischungskammer 2 sind beispielsweise als Schläuche ausgebildet. Sie sind mit Durchläufen 14 und 15 durch einen auf dem Deckel 5 des Behälters 1 installierten Rohransatz 16 und gemäß Fig. 1 durch einen auf dem Boden 3 oder auf dem Deckel 4 der Vermischungskammer 2 nicht dargestellten, installierten Rohransatz 17 gleichzeitig an einen Eingangsrohransatz 18 des Korrektursystems der Körperflüssigkeit angeschlossen. Dabei ist der Rohransatz 17 unter der Voraussetzung des Vorhandenseins der Möglichkeit der Einleitung der Körperflüssigkeit in die Vermischungskammer 2 unter einem Winkel von 10-80° zur Bodenfläche 3 oder zum Deckel 5 oder beispielsweise zur Wand 6 zwecks Sicherung der Torsion der Strömung dieser Flüssigkeit und deren bessere Vermischung mit dem magnetisch gesteuerten Sorbens installiert. Neben der auf dem Boden 3 der Vermischungskammer 2 befestigten Stirnfläche in der Seitenwand des Behälters 1 für das magnetisch gesteuerte Sorbens ist ein Durchlauf 19 vorgesehen, der für die Zufuhr des magnetisch gesteuerten Sorbens in die Vermischungskammer 2 bestimmt ist.

Der Durchlauf 20 aus der Vermischungskammer 2 in die Ausscheidungskammer 7 und der Durchlauf 21 aus der Ausscheidungskammer 7 in ein Filtergerät 22 sind entsprechend installiert: der Durchlauf 20 ist in der Wand 6 als Zwischenkammertrennwand neben deren Halterung am Boden 3 der Vermischungskammer 2 unter einem Winkel von 10-60° zum Boden 10 der Ausscheidungskammer 7 und zur Wand 6 installiert. Der Durchlauf 21 ist im Oberteil der Wand 12 der Ausscheidungskammer 7 installiert. Dabei ist das Filtergerät 22 mit seinem Durchlauf 23 mit dem Ausgangsrohransatz 24 des Systems gekoppelt.

Zwecks Sicherung der Richtbewegung der Körperflüssigkeit vom Eingangsrohransatz 18 durch das System zum Ausgangsrohransatz 24 sind Rückschlagventile 25 in den Systemdurchläufen und Rohransätzen installiert.

Das Filtergerät 22 ist gemäß Fig. 2 als entsprechendes Gerät ausgebildet (s. beispielsweise das oben genannte Patent US 5 980 479), das nacheinander installiert einen Ultrafiltrator 26 und einen Auffänger 27 enthält (s. dasselbe Patent), die beide für die Reinigung der Körperflüssigkeit von einer Fremdflüssigkeit (beispielsweise von Wassertröpfchen oder Luftblasen) bestimmt sind. Auf einem Eingangsdurchlauf 28 und auf einem Umgehungsdurchlauf 29 des Ultrafiltrators 26 sind Ventile 30 installiert, die im Notfall das Auslösen des Ultrafiltrators 26 während des Betriebs des Korrektursystems der Körperflüssigkeit und dessen Abschalten ermöglichen. Der Umgehungsdurchlauf 29 ist dabei zwecks Sicherung des Systembetriebs in den Betrieb des abgeschalteten Ultrafiltrators 26 eingeführt.

Die Volumina der Innenräume der Vermischungskammer 2 und der Ausscheidungskammer 7 sind im Verhältnis von 1:1 oder 1:(0,1-0,9) oder (0,1-0,9):1 ausgewählt. Die Volumina der Innenräume der Vermischungskammer 2 und des Behälters 1 für das magnetisch gesteuerte Sorbens sind im Verhältnis von 1:(0,1-0,9) ausgewählt. Außerdem ist der Behälter 1 in der Vermischungskammer 2 zur Vermischung des magnetisch gesteuerten Sorbens mit einem Abstand von mindestens (I-100)d von der Seitenwand 11 dieser Kammer und von mindestens (10-100)d von der Wand 6 zwischen der Vermischungskammer 2 und der Ausscheidungskammer 7 installiert, wobei d der Innendurchmesser des Durchlaufs 15 ist, der den Eingangsrohransatz 18 des Systems mit dem Innenraum der Vermischungskammer 2 zur Vermischung des magnetisch gesteuerten Sorbens verbindet. In dem beschriebenen Beispiel beträgt d=5-15mm.

Dabei sind die Innendurchmesser der der Rohransätze 16 und 17, die in die Vermischungskammer 2 und in den Behälter 1 für das magnetisch gesteuerte Sorbens hineingehen, im Verhältnis von d/d₁=V/V₁ ausgewählt, wobei d der Innendurchmesser des in die Vermischungskammer 2 hineingehenden Rohransatzes 17 ist, d₁ der Innendurchmesser des in den Behälter 1 hineingehenden Rohransatzes 16, V das Volumen der Vermischungskammer 2 und V₁ das Volumen des Behälters 1 für das magnetisch gesteuerte Sorbens. In dem beschriebenen Beispiel beträgt V₁=5-50ml.

Außerdem ist ein Durchlauf 19 aus dem Behälter 1 für das magnetisch gesteuerte Sorbens in die Vermischungskammer 2 beispielsweise im Unterteil der Seitenwand des Behälters 1 mit einem Abstand von (0,5-50)d vom Boden der Kammer installiert, wobei d der Innendurchmesser des Durchlaufs 19 ist. Ein Durchlauf 20 ist zwischen der Vermischungskammer 2 und der Ausscheidungskammer 7 in der Wand 6 mit einem Abstand von (0,5-50)d vom Boden 3 der Kammer 2 unter einem Winkel von 10-60° zur Fläche der Wand 6 und zum Boden 10 installiert, wobei d der Innendurchmesser des Durchlaufs 20 ist. Ein Durchlauf 21 aus der Ausscheidungskammer 7 ist im Obenteil der Seitenwand 12 der Ausscheidungskammer 7 mit einem Abstand von (0,5-50)d vom Deckel 8 installiert, hierbei ist d der Innendurchmesser des Durchlaufs 21. Im beschriebenen Beispiel sind die Durchmesser von allen Durchläufen 14, 15, 19, 20, 21, 23, 28 und 29 gleich.

Der Antrieb für die Volumenveränderung der Vermischungskammer 2 und der Ausscheidungskammer 7 und des Behälters 1 für das magnetisch gesteuerte Sorbens (nicht dargestellt) ist beispielsweise als Elektromotor ausgebildet (nicht dargestellt). Dieser Elektromotor ist mit dem Deckel 4 sowie mit dem Deckel 8 beispielsweise durch einen Drehzahlminderer mit Kurventrieb (nicht dargestellt) verbunden, oder der Antrieb für die Voluminaveränderung ist als Scheibchen 31 ausgebildet, das an der Abtriebswelle des Drehzahlminderers beispielsweise unter einem Winkel von 30-45° zur Wellenachse (nicht dargestellt) befestigt ist. Das Scheibchen 31 wirkt bei der Wellendrehung abwechselnd mit den Deckeln der Kammer zusammen.

Der Antrieb kann auch als mit dem Deckel gekoppelter Kurventrieb (nicht dargestellt) ausgebildet werden, der bei dessen Funktion die Handsteuerung einer Bedienungsperson ermöglicht, oder dieser Antrieb ermöglicht die Handeinwirkung der Bedienungsperson unmittelbar auf den Deckel.

Dabei ist die Stelle über der geriffelten Seitenwand 11 der Vermischungskammer 2 oder/und die Stelle über der geriffelten Seitenwand 12 der Ausscheidungskammer 7 als Stellort der Einwirkung auf den Deckel ausgewählt.

Wenn die bauliche Ausbildung des Bodens 3 der Vermischungskammer 2 und des Bodens 10 der Ausscheidungskammer 7 eine Drehungsmöglichkeit vorsieht, so ist dieser Boden 10 an der Zwischenkammertrennwand (an der Wand 6) mit Gelenken 32 befestigt (s. Fig. 5), die die Drehungsmöglichkeit jedes Bodens 3, 10 in der Fläche der Deckeldrehung jeder entsprechenden Kammer ermöglichen. Dabei sind zwecks des Ausschlusses einer unbefugten Drehung des Bodens die Gelenke 32 mit Sicherheitsschrauben (nicht dargestellt) ausgestattet.

Die mit der Vermischungskammer 2 und mit der Ausscheidungskammer 7 gebildete Gehäusekonfiguration kann im Grundriss beispielsweise entweder als Rechteck mit gerundeten Winkeln (nicht dargestellt) oder als Kreis (s. Fig. 6), als Oval (s. Fig. 7) oder als Ziffer 8 (s. Fig. 8) dargestellt werden.

### Das Korrektursystem der Körperflüssigkeit arbeitet folgendermaßen:

Die schrittweise, wechselhafte Einwirkung des Antriebs auf den Deckel 4 der Vermischungskammer 2 und auf den Deckel 8 der Ausscheidungskammer 7, deren Einwirkungsrhythmus beispielsweise von der Umdrehungszahl des Scheibchens 31 oder von der Häufigkeit des Drucks einer Bedienungsperson auf die Deckel abhängig ist, wechselt mit demselben Rhythmus die Volumina dieser Kammern sowie des Behälters 1, der im Innenraum der Vermischungskammer 2 installiert ist. Diese Volumenänderung wechselt dementsprechend (erhöht sich bei der Volumenabnahme und vermindert sich bei deren Zunahme) den Druck in den Kammern und im Behälter für das magnetisch gesteuerte Sorbens. Infolgedessen erfolgt ein periodisches Einsaugen der entsprechenden Flüssigkeit ins Korrektursystem oder das Auslassen dieser Flüssigkeit nach der Bearbeitung (dementsprechend in das Blutgefäßsystem eines Patienten oder in einen Sonderbehälter). Das Korrektursystem ist beispielsweise mit dem Blutgefäßsystem eines Patienten oder einfach mit dem Behälter (nicht dargestellt) mit der Körperflüssigkeit verbunden.

Dabei strömt die Körperflüssigkeit, z.B. Blut, aus der Blutader des Patienten in den Behälter 1, der vorher mit dem magnetisch gesteuerten Sorbens gefüllt war, und in die Vermischungskammer 2 durch entsprechende Durchläufe infolge der Antriebseinwirkung ein. Der Antrieb ist auf eine Zunahme des Volumina des Behälters 1 und der Vermischungskammer 2 in solcher Menge gerichtet, die dem Veränderungswert des entsprechenden Volumina verhältnisgleich ist.

Das in den Behälter 1 einströmende Blut bildet eine entsprechende Suspension mit dem dort vorhandenen magnetisch gesteuerten Sorbens, wobei die der Volumenveränderung des Behälters 1 entsprechende Suspensionsmenge (die Volumenveränderung wird durch die Antriebseinwirkung ausgelöst) durch den Durchlauf 19 in die Vermischungskammer 2 einströmt, in der das magnetisch gesteuerte Sorbens mit dieser Suspension vermischt wird und mit dem in diese Kammer eingeströmten Blut zusammenwirkt, wobei es entsprechende unerwünschte Fremdstoffe aufsaugt (s. beispielsweise die oben genannte WO 94 213 10 A1).

Zur intensiven Durchmischung des Bluts in der Vermischungskammer 2 mit dem magnetisch gesteuerten Sorbens trägt die Torsion der einfließenden Flüssigkeit infolge der Einleitung des Bluts in die Kammer unter dem angegebenen Winkel zum Boden 3 und zu den Wänden 6 und 11 bei.

Es sei bemerkt, dass ein Teil der Körperflüssigkeit, die in den Behälter 1 zur Suspensionsbildung mit dem magnetisch gesteuerten Sorbens einströmt, mit diesem Sorbens ebenfalls zusammenwirkt, aber die Konzentration des magnetisch gesteuerten Sorbens in dieser Suspension und die mit dieser Menge des magnetisch gesteuerten Sorbens verbundene Heilwirkung übersteigen bedeutend die Verluste, die infolge dieser Zusammenwirkung entstehen.

Bei der Volumenabnahme in der Vermischungskammer 2 und bei der Volumenzunahme in der Ausscheidungskammer 7 strömt das Gemisch des gereinigten Bluts mit dem magnetisch gesteuerten Sorbens durch den Durchlauf 20 in die Ausscheidungskammer 7, in der es unter der Einwirkung des Magnetfelds des magnetisch gesteuerten Sorbens im Bereich des Vorhandenseins der Magnete 13 ausgeschieden wird. Das gereinigte Blut fließt bei weiterer Volumenabnahme der Ausscheidungskammer 7 durch den Durchlauf 21 in das Filtergerät 22, wonach dieses Blut in das Blutgefäßsystem eines Patienten eingeleitet werden kann. Falls der Druck im System für die Strömung der Körperflüssigkeit durch das Filtergerät 22 unzureichend ist, wird beispielsweise eine auf dem Ausgangsdurchlauf 23 des Systems installierte Pumpe (nicht dargestellt) des peristaltischen Typs eingesetzt.

Das vorgeschlagene Korrektursystem für die Körperflüssigkeit ermöglicht eine qualitative Reinigung der Körperflüssigkeit ohne Hinzuziehung von zusätzlichen Reagenzien, beispielsweise durch den Einsatz eines magnetisch gesteuerten Sorbens ohne physiologische Lösung. Außerdem ermöglicht es, die Abmessungen des Korrektursystems bedeutend kleiner zu halten, wobei die nützlichen Volumina der Kammern und des Behälters nicht vermindert werden. Das Korrektursystem ermöglicht es, die Konstruktion zu vereinfachen, wobei es praktisch die Verwendung von Einweggeräten möglich macht, so dass das vorliegende Korrektursystem der Körperflüssigkeit nicht nur für die Klinikbehandlung, sondern auch für die ambulante Behandlung und bei der Nothilfeausübung, beispielsweise in der Unfallmedizin, einsetzbar ist.

### Bezugszeichenliste

- 1 -: Behälter zur Unterbringung der zu reinigenden Körperflüssigkeit
- 2 -: Vermischungskammer
- 3 -: Boden der Vermischungskammer 2
- 4 -: Deckel der Vermischungskammer 2
- 5 -: Deckel zum Überdecken des Deckels 4
- 6 -: Wand zwischen Vermischungskammer 2 und Ausscheidungskammer 7
- 7 -: Ausscheidungskammer
- 8 -: Deckel der Ausscheidungskammer 7
- 9 -: Gelenk an der Wand 6
- 10 -: Boden der Ausscheidungskammer 7
- 11 -: äußere Seitenwand der Vermischungskammer 2
- 12 -: äußere Seitenwand der Ausscheidungskammer 7
- 13 -: Magnete
- 14 -: Durchlauf zum Rohransatz 16
- 15 -: Durchlauf zum Rohransatz 17
- 16 -: Rohransatz am Deckel 5 des Behälters 1
- 17 -: Rohransatz im Boden 3 der Vermischungskammer 2
- 18 -: Eingangsrohransatz für die Rohransätze 16 und 17
- 19 -: Durchlauf in der Seitenwand des Behälters 1
- 20 -: Durchlauf in der Wand 6 zwischen Vermischungskammer 2 und Ausscheidungskammer 7
- 21 -: Durchlauf aus der Ausscheidungskammer 7 zu einem Filtergerät 22
- 22 -: Filtergerät
- 23 -: Durchlauf vom Durchlauf 21 zum Filtergerät 22
- 24 -: Ausgangsrohransatz des Filtergeräts 22
- 25 -: Rückschlagventil
- 26 -: Ultrafiltrator
- 27 -: Auffänger
- 28 -: Eingangsdurchlauf zum Ultrafiltrator 26
- 29 -: Umgehungsdurchlauf
- 30 -: Ventile
- 31 -: Scheibchen als Antrieb zur Voluminaveränderung
- 32 -: Gelenk an der Wand 6

## Patentansprüche

1. Korrektursystem der Körperflüssigkeit mit
- luftdichten, mit Rückschlagventilen (25) gekoppelten Durchläufen (16, 17, 19, 20, 21), wobei die Rückschlagventile (25) so installiert sind, dass sie ein Durchströmen der Körperflüssigkeit von einem Eingangsrohransatz (18) zu einem Ausgangsrohransatz (24) ermöglichen,
- einer Vermischungskammer (2) zur Vermischung eines magnetisch gesteuerten Sorbens mit der Körperflüssigkeit,
- einer Ausscheidungskammer (7) zur Ausscheidung dieses magnetisch gesteuerten Sorbens aus dieser Körperflüssigkeit,
- einem luftdichten Behälter (1) für das magnetisch gesteuerte Sorbens und
- einem Filtergerät (22), das mit einem Durchlauf (21) der Ausscheidungskammer (7) und dem Ausgangsrohransatz (24) des Systems gekoppelt ist,
- die Vermischungskammer (2) derart ausgebildet ist, dass ihr Volumen veränderbar und mit einem entsprechenden Antrieb ausgestattet ist,
**dadurch gekennzeichnet, dass**
- die Ausscheidungs Kammer sowie der Behälter für das magnetische Sorbens derart ausgebildet sind, dass ihre Volumina veränderbar und mit einem entsprechenden antrieb ausgestattet sind,
- die Vermischungskammer (2) und die Ausscheidungskammer (7) als Behälter ausgebildet sind, die fest miteinander gekoppelt sind,
- die Vermischungskammer 82) und die Ausscheidungskammer (7) einen gemeinsamen Deckel (4) sowie eine gemeinsame, auf dem Boden (3) befestigte Wand (6) als Zwischenkammertrennwand zur Ausscheidungskammer (7) aufweisen,
- die Innenräume der Vermischungskammer (2) und der Ausscheidungskammer (7) durch einen Durchlauf (20) in der Wand (6) miteinander verbunden sind,
- an den anderen Seitenwänden (11, 12) der Vermischungskammer (2) und der Ausscheidungskammer (7) Rillen vorgesehen sind, die entsprechende Faltrohre bilden,
- die Deckel (4, 8) der Vermischungskammer (2) und der Ausscheidungskammer (7) zur Drehung über Achsen von Gelenken (9) an der Wand (6) angelenkt sind,
- der Behälter (1) für das magnetisch gesteuerte Sorbens innerhalb der Vermischungskammer (2) für die Vermischung mit der Körperflüssigkeit installiert und mit einer als Zylinderfaltrohr geriffelten Seitenfläche ausgebildet ist,
- eine Stirnfläche dieses Zylinderfaltrohrs auf dem Boden (3) der Vermischungskammer (2) befestigt ist,
- an der anderen Stirnfläche dieses Zylinderfaltrohrs ein Deckel (5) installiert ist, der auf dem Deckel (4) der Vermischungskammer (2) befestigt ist,
- Magnete (13) auf dem Boden (10) der Ausscheidungskammer (7) aufgestellt sind und
- der Eingangsrohransatz (18) des Systems gleichzeitig mit den Innenräumen der Vermischungskammer (2), der Ausscheidungskammer (7) und des Behälters (1) für das magnetisch gesteuerte Sorbens verbunden ist, wobei der Behälter (1) mit dem Innenraum der Vermischungskammer (2) gekoppelt ist.

2. Korrektursystem nach Anspruch 1,
**dadurch gekennzeichnet,**
**dass** die Deckel (4, 8) der Vermischungskammer (2) und der Ausscheidungskammer (7) in einer Fläche liegen.

3. Korrektursystem nach Anspruch 1,
**dadurch gekennzeichnet,**
**dass** die Deckel (4, 8) der Vermischungskammer (2) und der Ausscheidungskammer (7) als im Schnitt V-förmiges Winkelprofil miteinander gekoppelt sind.

4. Korrektursystem nach einem der Ansprüche 1 bis 3,
**dadurch gekennzeichnet,**
**dass** das mit der Vermischungskammer (2) und mit der Ausscheidungskammer (7) gebildete Gehäuse im Grundriss als Rechteck mit gerundeten Winkeln, als Kreis, Oval oder Ziffer 8 dargestellt ist.

5. Korrektursystem nach einem der Ansprüche 1 bis 3,
**dadurch gekennzeichnet,**
**dass** die Böden (4, 8) der Vermischungskammer (2) und der Ausscheidungskammer (7) fest mit der Wand (6) gekoppelt sind.

6. Korrektursystem nach einem der Ansprüche 1 bis 3,
**dadurch gekennzeichnet,**
**dass** in der Ecke der Wand (6) ein Gelenk (9) der Deckel (4, 8) installiert ist.

7. Korrektursystem nach einem der Ansprüche 1 bis 3,
**dadurch gekennzeichnet,**
**dass** die Böden (3, 10) der Vermischungskammer (2) und der Ausscheidungskammer (7) mit der Wand (6) derart gekoppelt sind, dass sie in der Fläche der Deckeldrehung ebenfalls drehbar sind.

8. Korrektursystem nach einem der Ansprüche 1 bis 3,
**dadurch gekennzeichnet,**
**dass** die Volumina der Innenräume der Vermischungskammer (2) und der Ausscheidungskammer (7) im Verhältnis von 1:1 oder von 1:(0,1-0,9) oder von (0,1-0,9):1 entsprechend ausgewählt sind und die Volumina der Innenräume der Vermischungskammer (2) und des Behälters (1) für das magnetisch gesteuerte Sorbens im Verhältnis von 1:(0,1-0,9) ausgewählt sind.

9. Korrektursystem nach Anspruch 1,
**dadurch gekennzeichnet,**
**dass** der Behälter (1) für das magnetisch gesteuerte Sorbens in der Vermischungskammer (2) für die Vermischung des magnetisch gesteuerten Sorbens mit einem Abstand von mindestens (I-100)d von der Seitenwand (11) dieser Vermischungskammer (2) und von mindestens (10-100)d von der Wand (6) zwischen der Vermischungskammer (2) und der Ausscheidungskammer (7) installiert ist, wobei d den Innendurchmesser des Durchlaufs (15) darstellt, der den Eingangsrohransatz (16) des Systems mit dem Innenraum der Vermischungskammer (2) verbindet.

10. Korrektursystem nach Anspruch 9,
**dadurch gekennzeichnet,**
**dass** der Durchlauf (17) vom Eingangsrohransatz (18) in die Vermischungskammer (2) entweder durch den Boden (3) oder durch den Deckel (4) der Vermischungskammer (2) eingeleitet ist.

11. Korrektursystem nach Anspruch 10,
**dadurch gekennzeichnet,**
**dass** der Durchlauf (17) vom Eingangsrohransatz (18) in die Vermischungskammer (2) unter einem Winkel von 10-80° zur Bodenfläche oder zur Kammerdeckelfläche und zur Vertikalen eingeleitet ist.

12. Korrektursystem nach Anspruch 9,
**dadurch gekennzeichnet,**
**dass** der Durchlauf (16) vom Eingangsrohransatz in den Behälter (1) für das magnetisch gesteuerte Sorbens durch den Deckel (5) des Behälters (1) eingeleitet ist, und der Durchlauf (19) aus dem Behälter (1) für das magnetisch gesteuerte Sorbens in die Vermischungskammer (2), im Unterteil der Seitenwand des Behälters (1) und der Vermischungskammer (2), mit einem Abstand von (0,5-50)d vom Boden (3) der Vermischungskammer (2) installiert ist, wobei d der Durchmesser des Durchlaufs (16) ist.

13. Korrektursystem nach Anspruch 9,
**dadurch gekennzeichnet,**
**dass** der Durchlauf (20) zwischen der Vermischungskammer (2) und der Ausscheidungskammer (7) in der Wand (6) zwischen der Vermischungskammer (2) und der Ausscheidungskammer (7) mit einem Abstand von (0,5-50)d vom Boden (3, 10) installiert ist, wobei d der Durchmesser des Durchlaufs (20) ist.

14. Korrektursystem nach Anspruch 9,
**dadurch gekennzeichnet,**
**dass** der Durchlauf (20) zwischen der Vermischungskammer (2) und der Ausscheidungskammer (7) in der Wand (6) zwischen der Vermischungskammer (2) und der Ausscheidungskammer (7) unter einem Winkel von 10-60° zum Boden (10) der Ausscheidungskammer und zur Wand (6) installiert ist.

15. Korrektursystem nach Anspruch 9,
**dadurch gekennzeichnet,**
**dass** der Durchlauf (21) aus der Ausscheidungskammer (7) entweder im Deckel (8) oder im Oberteil der Seitenwand (12) der Ausscheidungskammer (7) mit einem Abstand von (0,5-50)d vom Deckel (10) installiert ist, wobei d der Durchmesser des Durchlaufs (21) ist.

16. Korrektursystem nach Anspruch 1,
**dadurch gekennzeichnet,**
**dass** die Magnete (13) entweder innerhalb oder außerhalb der Ausscheidungskammer (7) aufgestellt sind oder dass sie entweder innerhalb oder außerhalb der Ausscheidungskammer (7) aufgestellt und auf dem Boden (10) der Ausscheidungskammer (7) befestigt sind.

17. Korrektursystem nach Anspruch 1,
**dadurch gekennzeichnet,**
**dass** der Antrieb für die Voluminaveränderung der Vermischungskammer (2) und der Ausscheidungskammer (7) und des Behälters (1) für das magnetisch gesteuerte Sorbens vorzugsweise als Elektromotor ausgebildet ist, der mit dem Deckel (5) vorzugsweise durch einen Drehzahlminderer mit Kurventrieb verbunden ist, oder dass der Antrieb als an der Abtriebswelle des Drehzahlminderers unter einem Winkel von 30-45° zur Wellenachse befestigter Drehzahlminderer ausgebildet ist, der bei der Wellendrehung abwechselnd mit den Deckeln (4, 8) der Vermischungskammer (2) und der Ausscheidungskammer (7) zusammenwirkt.

18. Korrektursystem nach Anspruch 1,
**dadurch gekennzeichnet,**
**dass** der Antrieb für die Voluminaveränderung der Vermischungskammer (2), der Ausscheidungskammer (7) und des Behälters (1) als mit dem Deckel (5) gekoppelter Kurventrieb ausgebildet ist, bei dessen Funktion die Handsteuerung einer Bedienungsperson möglich ist.

19. Korrektursystem nach Anspruch 1,
**dadurch gekennzeichnet,**
**dass** der Antrieb derart ausgebildet ist, dass die Handeinwirkung einer Bedienungsperson unmittelbar auf den Deckel (5) möglich ist.

20. Korrektursystem nach einem der Ansprüche 1, 17-19,
**dadurch gekennzeichnet,**
**dass** die Stelle über der geriffelten Seitenwand (11) der Vermischungskammer (2) oder über der geriffelten Seitenwand (12) der Ausscheidungskammer (7) als Stellort der Einwirkung auf den Deckel (5) ausgewählt ist.

21. Korrektursystem nach Anspruch 9,
**dadurch gekennzeichnet,**
**dass** die Durchmesser der in die Vermischungskammer (2) und in den Behälter (1) für das magnetisch gesteuerte Sorbens eingehenden Durchläufe (16, 17) im Verhältnis von d/d₁=V/V₁ ausgewählt sind, wobei d der Innendurchmesser des in die Vermischungskammer (2) eingehenden Durchlaufs (17), d₁ der Innendurchmesser des in den Behälter (1) für das magnetisch gesteuerte Sorbens eingehenden Durchlaufs (16), V das Volumen der Vermischungskammer (2) und V₁ das Volumen des Behälters (1) für das magnetisch gesteuerte Sorbens ist.

22. Korrektursystem nach Anspruch 1,
**dadurch gekennzeichnet,**
**dass** die Wände des Behälters (1) für das magnetisch gesteuerte Sorbens, der Vermischungskammer (2) und der Ausscheidungskammer (7), die Wand (6), der Deckel (5) und der Boden (3, 10) vorzugsweise aus Polyurethan bestehen.

23. Korrektursystem nach Anspruch 22,
**dadurch gekennzeichnet,**
**dass** die Wellung im Behälter für das magnetisch gesteuerte Sorbens und in der Vermischungskammer (2) sowie in der Ausscheidungskammer (7) an (0,5-0,95)% der Höhe der entsprechenden Wände aufgetragen ist.

## Claims

1. A correction system for body fluid, having
- airtight passages (16, 17, 19, 20, 21), coupled with check valves (25), the check valves (25) being installed such that they enable a flow of the body fluid from an inlet tube attachment (18) to an outlet tube attachment (24),
- a mixing chamber (2) for mixing a magnetically controlled sorbent with the body fluid,
- a settling chamber (7) for settling this magnetically controlled sorbent out of this body fluid,
- an airtight container (1) for the magnetically controlled sorbent, and
- a filter device (22), which is coupled with a passage (21) of the settling chamber (7) and with the outlet tube attachment (24) of the system,
- the mixing chamber (2) is embodied such that its volume is variable and is equipped with a corresponding drive mechanism,
**characterized in that**
- the settling chamber and the container for the magnetic sorbent are embodied such that their volumes are variable and are equipped with a corresponding drive mechanism;
- the inner chambers of the mixing chamber (2) and of the settling chamber (7) communicate with one another through a passage (20) in the wall (6);
- on the other side walls (11, 12) of the mixing chamber (2) and of the settling chamber (7), grooves are provided, which form corresponding accordion-folded tubes;
- the caps (4, 8) of the mixing chamber (2) and of the settling chamber (7) are pivotably connected for rotation via axes of joints (9) on the wall (6);
- the container (1) for the magnetically controlled sorbent is installed inside the mixing chamber (2) for the mixing with the body fluid and is embodied as a cylindrical accordion-folded tube with a corrugated side face; of the mixing chamber (2);
- on the other end face of this cylindrical accordion-folded tube, a cap (5) is installed which is secured to the cap (4) of the mixing chamber (2);
- magnets (13) are placed on the bottom (10) of the settling chamber (7); and
- the inlet tube attachment (18) of the system is simultaneously coupled with the inner chambers of the mixing chamber (2), of the settling chamber (7), and of the container (1) for the magnetically controlled sorbent, and the container (1) is coupled with the inner chamber of the mixing chamber (2).

2. The correction system as defined by claim 1,
**characterized in that**
the caps (4, 8) of the mixing chamber (2) and of the settling chamber (7) are located in one surface.

3. The correction system as defined by claim 1,
**characterized in that**
the caps (4, 8) of the mixing chamber (2) and of the settling chamber (7) are coupled to one another in the form of an angle profile that is V-shaped in section.

4. The correction system as defined by one of claims 1 through 3,
**characterized in that**
the housing formed by the mixing chamber (2) and by the settling chamber (7) is represented in outline as a rectangle with rounded corners, as a circle, as an oval, or as a figure 8.

5. The correction system as defined by one of claims 1 through 3,
**characterized in that**
the bottoms (4, 8) of the mixing chamber (2) and of the settling chamber (7) are solidly coupled to the wall (6).

6. The correction system as defined by one of claims 1 through 3,
**characterized in that**
a joint (9) of the caps (4, 8) is installed in the corner of the wall (6).

7. The correction system as defined by one of claims 1 through 3,
**characterized in that**
the bottoms (3, 10) of the mixing chamber (2) and of the settling chamber (7) are coupled to the wall (6) in such a way that they are likewise rotatable in the area of the cap rotation.

8. The correction system as defined by one of claims 1 through 3,
**characterized in that**
the volumes of the inner chambers of the mixing chamber (2) and of the settling chamber (7) are suitably selected in the ratio of 1:1 or of 1:(0.1-0.9) or of (0.1-0.9):1, and the volumes of the inner chambers of the mixing chamber (2) and of the container (1) for the magnetically controlled sorbent are selected in the ratio of 1:(0.1-0.9).

9. The correction system as defined by claim 1,
**characterized in that**
the container (1) for the magnetically controlled sorbent is installed in the mixing chamber (2) for the mixing of the magnetically controlled sorbent with a spacing of at least (1-100)d from the side wall (11) of this mixing chamber (2) and of at least (10-100)d from the wall (6) between the mixing chamber (2) and the settling chamber (7), where d represents the inside diameter of the passage (15) that connects the inlet tube attachment (16) of the system with the inner chamber of the mixing chamber (2).

10. The correction system as defined by claim 9,
**characterized in that**
the passage (17) from the inlet tube attachment (18) into the mixing chamber (2) is introduced either through the bottom (3) or through the cap (4) of the mixing chamber (2).

11. The correction system as defined by claim 10,
**characterized in that**
the passage (17) from the inlet tube attachment (18) into the mixing chamber (2) is introduced at an angle of 10-80° to the bottom face or to the chamber cap face and to the vertical.

12. The correction system as defined by claim 9,
**characterized in that**
the passage (16) is introduced from the inlet tube attachment into the container (1) for the magnetically controlled sorbent through the cap (5) of the container (1), and the passage (19) from the container (1) for the magnetically controlled sorbent into the mixing chamber (2), in the lower part of the side wall of the container (1) and of the mixing chamber (2), is installed with a spacing of (0.5-50)d from the bottom (3) of the mixing chamber (2), where d is the diameter of the passage (16).

13. The correction system as defined by claim 9,
**characterized in that**
the passage (20) between the mixing chamber (2) and the settling chamber (7) is installed in the wall (6) between the mixing chamber (2) and the settling chamber (7) with a spacing of (0.5-50)d from the bottom (3, 10), where d is the diameter of the passage (20).

14. The correction system as defined by claim 9,
**characterized in that**
the passage (20) between the mixing chamber (2) and the settling chamber (7) is installed in the wall (6) between the mixing chamber (2) and the settling chamber (7) at an angle of 10-60° from the bottom (10) of the settling chamber and from the wall (6).

15. The correction system as defined by claim 9,
**characterized in that**
the passage (21) from the settling chamber (7) is installed either in the cap (8) or in the upper part of the side wall (12) of the settling chamber (7) with a spacing of (0.5-50)d from the cap (10), where d is the diameter of the passage (21).

16. The correction system as defined by claim 1,
**characterized in that**
the magnets (13) are placed either inside or outside the settling chamber (7); or that they are placed either inside or outside the settling chamber (7) and are secured to the bottom (10) of the settling chamber (7).

17. The correction system as defined by claim 1,
**characterized in that**
the drive mechanism for changing the volume of the mixing chamber (2) and of the settling chamber (7) and of the container (1) for the magnetically controlled sorbent is preferably embodied as an electric motor, which is connected to the cap (5), preferably by an rpm reducer with a cam drive mechanism; or that the drive mechanism is embodied as an rpm reducer, secured to the power takeoff shaft of the rpm reducer at an angle of 30-45° to the shaft axis, which rpm reducer upon the shaft rotation cooperates in alternation with the caps (4, 8) of the mixing chamber (2) and of the settling chamber (7).

18. The correction system as defined by claim 1,
**characterized in that**
the drive mechanism for changing the volume of the mixing chamber (2), of the settling chamber (7), and of the container (1) is embodied as a cam drive mechanism coupled to the cap (5), upon the function of which drive mechanism manual control by a human operator is possible.

19. The correction system as defined by claim 1,
**characterized in that**
the drive mechanism is embodied such that manual action by a human operator directly on the cap (5) is possible.

20. The correction system as defined by one of claims 1, 17-19,
**characterized in that**
the location above the corrugated side wall (11) of the mixing chamber (2) or above the corrugated side wall (12) of the settling chamber (7) is selected as the site of the action on the cap (5).

21. The correction system as defined by claim 9,
**characterized in that**
the diameter of the passages (16, 17), entering into the mixing chamber (2) and into the container (1) for the magnetically controlled sorbent, are selected in the ratio of d/d₁=V/V₁, where d is the inside diameter of the passage (17) entering the mixing chamber (2), d₁ is the inside diameter of the passage (16) entering the container (1) for the magnetically controlled sorbent, V is the volume of the mixing chamber (2), and V₁ is the volume of the container (1) for the magnetically controlled sorbent.

22. The correction system as defined by claim 1,
**characterized in that**
the walls of the container (1) for the magnetically controlled sorbent, of the mixing chamber (2), and of the settling chamber (7), and the wall (6), the cap (5) and the bottoms (3, 10) preferably comprise polyurethane.

23. The correction system as defined by claim 22,
**characterized in that**
the corrugation in the container for the magnetically controlled sorbent and in the mixing chamber (2) as well as in the settling chamber (7) is applied at (0.5-0.95)% of the height of the corresponding walls.

## Revendications

1. Système de correction d'un liquide corporel, avec
- des passages (16, 17, 19, 20, 21) étanches à l'air, couplés à des clapets antiretour (25), sachant que les clapets antiretour (25) sont installés de telle sorte qu'ils permettent l'écoulement du liquide corporel depuis une tubulure d'entrée (18) vers une tubulure de sortie (24),
- une chambre de mélangeage (2) pour mélanger au liquide corporel un adsorbant commandé magnétiquement,
- une chambre de séparation (7) pour éliminer du liquide corporel par séparation cet adsorbant commandé magnétiquement,
- un récipient étanche à l'air (1) pour l'adsorbant commandé magnétiquement, et
- un appareil de filtration (22), qui est couplé à un passage (21) de la chambre de séparation (7) et à la tubulure de sortie (24) du système,
- la chambre de mélangeage (2) étant conçue de telle sorte que son volume est variable et elle est équipée d'un entraînement correspondant,
**caractérisé en ce que**
- la chambre de séparation ainsi que le récipient pour l'adsorbant magnétique sont conçus de telle sorte que leurs volumes sont variables et ils sont équipés d'un entraînement correspondant,
- la chambre de mélangeage (2) et la chambre de séparation (7) sont réalisées sous la forme de récipients qui sont fixement couplés entre eux,
- la chambre de mélangeage (2) et la chambre de séparation (7) présentent un couvercle commun (4) ainsi qu'une paroi commune (6), fixée sur le fond (3), comme paroi de séparation intermédiaire vis-à-vis de la chambre de séparation (7),
- les espaces intérieurs de la chambre de mélangeage (2) et de la chambre de séparation (7) sont reliés entre eux par un passage (20) dans la paroi (6),
- des gorges, qui forment des soufflets tubulaires correspondants, sont prévues sur les autres parois latérales (11, 12) de la chambre de mélangeage (2) et de la chambre de séparation (7),
- les couvercles (4, 8) de la chambre de mélangeage (2) et de la chambre de séparation (7) sont articulés en rotation autour d'axes d'articulations (9) sur la paroi (6),
- le récipient (1) pour l'adsorbant commandé magnétiquement est installé à l'intérieur de la chambre de mélangeage (2) pour le mélangeage avec le liquide corporel, et il est réalisé avec une surface latérale cannelée sous forme de soufflet tubulaire cylindrique,
- une surface frontale de ce soufflet tubulaire cylindrique est fixée sur le fond (3) de la chambre de mélangeage (2),
- un couvercle (5), qui est fixé sur le couvercle (4) de la chambre de mélangeage (2), est installé sur l'autre surface frontale de ce soufflet tubulaire cylindrique,
- des aimants (13) sont mis en place sur le fond (10) de la chambre de séparation (7), et
- la tubulure d'entrée (18) du système est simultanément reliée aux espaces intérieurs de la chambre de mélangeage (2), de la chambre de séparation (7) et du récipient (1) pour l'adsorbant commandé magnétiquement, sachant que le récipient (1) est couplé à l'espace intérieur de la chambre de mélangeage (2).

2. Système de correction selon la revendication 1, **caractérisé en ce que** les couvercles (4, 8) de la chambre de mélangeage (2) et de la chambre de séparation (7) se trouvent sur un même plan.

3. Système de correction selon la revendication 1, **caractérisé en ce que** les couvercles (4, 8) de la chambre de mélangeage (2) et de la chambre de séparation (7) sont couplés entre eux en tant que profilé angulaire présentant en coupe une forme de V.

4. Système de correction selon l'une des revendications 1 à 3, **caractérisé en ce que** le boîtier formé par la chambre de mélangeage (2) et la chambre de séparation (7) présente en projection horizontale une forme de rectangle à angles arrondis, de cercle, d'ovale ou de huit.

5. Système de correction selon l'une des revendications 1 à 3, **caractérisé en ce que** les fonds (3, 10) de la chambre de mélangeage (2) et de la chambre de séparation (7) sont fixement couplés à la paroi (6).

6. Système de correction selon l'une des revendications 1 à 3, **caractérisé en ce qu'**une articulation (9) des couvercles (4, 8) est installée dans le coin de la paroi (6).

7. Système de correction selon l'une des revendications 1 à 3, **caractérisé en ce que** les fonds (3, 10) de la chambre de mélangeage (2) et de la chambre de séparation (7) sont couplés à la paroi (6) de telle sorte qu'ils peuvent eux aussi être tournés dans le plan de la rotation des couvercles.

8. Système de correction selon l'une des revendications 1 à 3, **caractérisé en ce que** les volumes des espaces intérieurs de la chambre de mélangeage (2) et de la chambre de séparation (7) sont adéquatement choisis dans le rapport de 1:1 ou de 1:(0,1-0,9) ou de (0,1-0,9):1, et les volumes des espaces intérieurs de la chambre de mélangeage (2) et du récipient (1) pour l'adsorbant commandé magnétiquement sont choisis dans le rapport de 1:(0,1-0,9).

9. Système de correction selon la revendication 1, **caractérisé en ce que** le récipient (1) pour l'adsorbant commandé magnétiquement est installé dans la chambre de mélangeage (2) pour le mélangeage de l'adsorbant commandé magnétiquement à une distance d'au moins (I-100)d de la paroi latérale (11) de cette chambre de mélangeage (2) et d'au moins (10-100)d de la paroi (6) entre la chambre de mélangeage (2) et la chambre de séparation (7), où d représente le diamètre intérieur du passage (15) qui relie la tubulure d'entrée (18) du système à l'espace intérieur de la chambre de mélangeage (2).

10. Système de correction selon la revendication 9, **caractérisé en ce que** le passage (17) menant de la tubulure d'entrée (18) à la chambre de mélangeage (2) est introduit soit à travers le fond (3), soit à travers le couvercle (4) de la chambre de mélangeage (2).

11. Système de correction selon la revendication 10, **caractérisé en ce que** le passage (17) menant de la tubulure d'entrée (18) à la chambre de mélangeage (2) est introduit sous un angle de 10 à 80° par rapport à la surface de fond ou par rapport à la surface du couvercle de la chambre et par rapport à la verticale.

12. Système de correction selon la revendication 9, **caractérisé en ce que** le passage (16) menant de la tubulure d'entrée au récipient (1) pour l'adsorbant commandé magnétiquement est introduit à travers le couvercle (5) du récipient (1), et le passage (19) quittant le récipient (1) pour l'adsorbant commandé magnétiquement pour aller dans la chambre de mélangeage (2) est installé dans la partie inférieure de la paroi latérale du récipient (1) et de la chambre de mélangeage (2) à une distance de (0,5-50)d du fond (3) de la chambre de mélangeage (2), où d est le diamètre du passage (16).

13. Système de correction selon la revendication 9, **caractérisé en ce que** le passage (20) entre la chambre de mélangeage (2) et la chambre de séparation (7) est installé dans la paroi (6) entre la chambre de mélangeage (2) et la chambre de séparation (7) à une distance de (0,5-50)d du fond (3, 10), où d est le diamètre du passage (20).

14. Système de correction selon la revendication 9, **caractérisé en ce que** le passage (20) entre la chambre de mélangeage (2) et la chambre de séparation (7) est installé dans la paroi (6) entre la chambre de mélangeage (2) et la chambre de séparation (7) sous un angle de 10 à 60° par rapport au fond (10) de la chambre de séparation et à la paroi (6).

15. Système de correction selon la revendication 9, **caractérisé en ce que** le passage (21) quittant la chambre de séparation (7) est installé soit dans le couvercle (8) soit dans la partie supérieure de la paroi latérale (12) de la chambre de séparation (7) à une distance de (0,5-50)d du couvercle (8), où d est le diamètre du passage (21).

16. Système de correction selon la revendication 1, **caractérisé en ce que** les aimants (13) sont mis en place soit à l'intérieur soit à l'extérieur de la chambre de séparation (7), ou **en ce qu'**ils sont mis en place soit à l'intérieur soit à l'extérieur de la chambre de séparation (7) et sont fixés sur le fond (10) de la chambre de séparation (7).

17. Système de correction selon la revendication 1, **caractérisé en ce que** l'entraînement pour la modification des volumes de la chambre de mélangeage (2), de la chambre de séparation (7) et du récipient (1) pour l'adsorbant commandé magnétiquement est réalisé de préférence sous forme de moteur électrique qui est relié au couvercle (5) de préférence par un réducteur de régime avec un mécanisme à cames, ou **en ce que** l'entraînement est réalisé sous la forme d'un réducteur de régime qui est fixé sur l'arbre de sortie du réducteur de régime sous un angle de 30 à 45° par rapport à l'axe de l'arbre et qui, lors de la rotation de l'arbre, coopère alternativement avec les couvercles (4, 8) de la chambre de mélangeage (2) et de la chambre de séparation (7).

18. Système de correction selon la revendication 1, **caractérisé en ce que** l'entraînement pour la modification des volumes de la chambre de mélangeage (2), de la chambre de séparation (7) et du récipient (1) est réalisé sous forme de mécanisme à cames qui est couplé au couvercle (5) et qui autorise la commande manuelle d'un opérateur pour son fonctionnement.

19. Système de correction selon la revendication 1, **caractérisé en ce que** l'entraînement est conçu de telle sorte qu'il permet l'action manuelle d'un opérateur directement sur le couvercle (5).

20. Système de correction selon l'une des revendications 1 et 17 à 19, **caractérisé en ce que** l'emplacement au-dessus de la paroi latérale striée (11) de la chambre de mélangeage (2) ou au-dessus de la paroi latérale striée (12) de la chambre de séparation (7) est choisi comme point d'action sur le couvercle (5).

21. Système de correction selon la revendication 9, **caractérisé en ce que** les diamètres des passages (16, 17) entrant dans la chambre de mélangeage (2) et dans le récipient (1) pour l'adsorbant commandé magnétiquement sont choisis dans le rapport d/d₁ = V/V₁, où d est le diamètre intérieur du passage (17) entrant dans la chambre de mélangeage (2), d₁ le diamètre intérieur du passage (16) entrant dans le récipient (1) pour l'adsorbant commandé magnétiquement, V le volume de la chambre de mélangeage (2) et V₁ le volume du récipient (1) pour l'adsorbant commandé magnétiquement.

22. Système de correction selon la revendication 1, **caractérisé en ce que** les parois du récipient (1) pour l'adsorbant commandé magnétiquement, de la chambre de mélangeage (2) et de la chambre de séparation (7), la paroi (6), le couvercle (5) et les fonds (3, 10) sont constitués de préférence de polyuréthane.

23. Système de correction selon la revendication 22, **caractérisé en ce que** l'ondulation dans le récipient pour l'adsorbant commandé magnétiquement et dans la chambre de mélangeage (2) ainsi que dans la chambre de séparation (7) est réalisée sur (0,5-0,95)% de la hauteur des parois correspondantes.
